# EUROPEAN PATENT SPECIFICATION

(11) **EP 4 049 701 B1**
(45) Date of publication and mention of the grant of the patent: **13.11.2024**
(21) Application number: 20893351.5
(22) Date of filing: 25.11.2020
(51) Int. Cl.: A61M 16/06, A61M 16/08

(54) **PATIENT INTERFACE DEVICE AND VENTILATION TREATMENT APPARATUS**
PATIENTENSCHNITTSTELLENVORRICHTUNG UND VENTILATIONSBEHANDLUNGSVORRICHTUNG
DISPOSITIF D'INTERFACE PATIENT ET APPAREIL DE TRAITEMENT DE VENTILATION

(30) Priority: 27.11.2019 CN 201911185587
(43) Date of publication of application: 31.08.2022
(73) Proprietor: BMC (Tianjin) Medical Co., Ltd., Tianjin 301700 (CN)
(72) Inventor: XU, Han, Tianjin 301700 (CN); CHEN, Yunjing, Tianjin 301700 (CN); ZHUANG, Zhi, Beijing 100041 (CN)
(74) Representative: Grünecker Patent- und Rechtsanwälte PartG mbB
(86) International application number: PCT/CN2020/131318
(87) International publication number: WO 2021/104280

(56) References cited:
- EP-A1- 2 522 275
- EP-A2- 3 338 847
- CN-A- 109 999 295
- CN-A- 109 999 295
- CN-A- 110 833 647
- CN-U- 203 898 886
- CN-U- 205 434 636
- CN-U- 211 327 569
- JP-A- 2019 193 686
- US-A- 3 603 313
- US-A1- 2014 158 127
- US-A1- 2018 280 650

## Description

### TECHNICAL FIELD

The present application relates to the field of ventilation treatment device, and particularly relates to a patient interface apparatus and a ventilation treatment device including the patient interface apparatus.

### BACKGROUND

Non-invasive positive pressure ventilation has been widely used in treatment of obstructive sleep apnea (OSA), chronic obstructive pulmonary disease (COPD) and other diseases. In the non-invasive positive pressure ventilation, a blower is used to provide a continuous positive airway pressure (CPAP) or a variable pressure ventilation to a patient's airway through a tube and a patient interface apparatus, instead of surgically inserting a tube in the patient's airway.

The patient interface apparatus of the non-invasive ventilation treatment is usually provided with a nasal oxygen catheter and types of breathing masks such as a nasal mask, an oral-nasal mask and a full-face mask and so forth. Either the nasal oxygen catheter or the breathing mask, a gas cavity which is connected with the mouth/nose of the patient is provided to supply gas to the patient. During the treatment process, due to water vapor is contained in both inhaled and exhaled gas by the patient, it is very easy to condense water in the gas cavity, and excessive condensed water may pose a potential threat to the patient.

Due to the patient interface apparatus on the current market is not provided with a function of automatically removing condensed water, it requires active intervention of medical staffs, which makes the apparatus inconvenient to use and increase labor intensity of the medical staffs at the same time. Therefore, it is necessary to provide a patient interface apparatus which is capable to automatically remove condensed water, thereby improving safety of ventilation treatment and reducing labor intensity of the medical staffs.

US 3603 313A discloses a throwaway sanitary collector of condensate carried in exhalant of a patient using an intermittent positive pressure breathing apparatus. The collector comprises a relatively thin plaque of fine gossamer, the outer rim portion of which is folded upwardly and sandwiched between a mass of highly absorbent material with a portion of its convolutions formed inwardly of the upturned edges of plaque and additional convolutions encircling the exterior of the gossamer.

CN 109 999 295 A discloses a patient interface device including a moisture outlet structure, which comprises an opening and an exchange layer. The opening is provided on the main body for communicating the gas chamber with the external environment. The exchanger layer covers the opening and is arranged to absorb moisture in the gas chamber and discharge it to the external environment.

US 2014/158127 A1 discloses a nasal cannula assembly comprising a face piece, wherein a liquid absorbent material can be disposed in an internal volume defined by the face piece that is operable to absorb the rainout droplets and wick it away along the entire circumference of the second end portion to a raindrop sink, such as a paper plug. However, this solution can only absorb a limited amount of moisture for a limited number of hours, before the rainout sink has to be dried or replaced.

US2018/280650 A1 discloses a conduit for use in a pressure support system for communicating a flow of pressurized gas to the airway of a patient includes a first strip of a first material disposed helically about a central longitudinal axis such that subsequent helical convolutions of the first strip are disposed adjacent each other, and a second strip of a second material disposed helically about the central longitudinal axis along the first strip. The second strip is coupled between the subsequent helical convolutions of the first strip so as to form a hollow conduit. The first material is structured to prevent the passage of fluids therethrough. The second material is structured to allow passage of a liquid therethrough while inhibiting passage of gases therethrough.

### SUMMARY

The invention is defined in the appended independent claim 1. Preferred embodiments are matter of the dependent claims.

The object of the present application is to provide a patient interface apparatus and a ventilation treatment device including the patient interface apparatus, to solve the problems as mentioned above, in particular, by removing the moisture in an easier and user-friendly way.

In order to implement the object mentioned above, one aspect of the present application provides a patient interface apparatus for a ventilation treatment device, wherein the patient interface apparatus comprises a main body and a first moisture-extraction part, wherein a gas cavity is defined inside the main body and configured to communicate with the patient's mouth and/or nose, the first moisture-extraction part is disposed on the main body and comprises a first part communicate with the gas cavity and a second part located outside the main body, the first moisture-extraction part is disposed to be capable to absorb moisture inside of the gas cavity and conduct the moisture to the second part through the first part. The first part is directly disposed inside the gas cavity and the second part is configured to evaporate the moisture to the external environment in use.

Optionally, the patient interface apparatus comprises a headband, the headband is configured to fix the main body to the patient's head, the first moisture-extraction part is formed as either a part of the headband or the entire headband.

Optionally, the moisture-extraction part comprises a fixed stacked water-absorbing layer and a waterproof layer, the waterproof layer is disposed close to a side of the headband for touching the patient's head.

Optionally, the water-absorbing layer is made of fabric or absorbent dressing.

Optionally, the waterproof layer is made of plastic or rubber.

Optionally, the patient interface apparatus is a nasal oxygen catheter, the main body comprises a nasal connector and an adapting connector installed on the nasal connector, the first moisture-extraction part is installed on the nasal connector, the gas cavity is jointly limited formed by the first part, the nasal connector and the adapting connector, the adapting connector is capable to pass gas from a gas source to the gas cavity.

Optionally, the nasal connector comprises an installation structure configured to install the adapting connector, the installation structure comprises a containing cavity with a bottom opening and a top wall and a rear wall which are configured to form the containing cavity, the first part is installed on an inner surface of the rear wall, the adapting connector comprises a shell with a top opening and a rear opening, the shell is sealed and installed in the containing cavity via the bottom opening of the installation structure, the gas cavity is formed by the shell, the top wall and the first part.

Optionally, the nasal connector comprises a nasal plug configured to insert a nasal cavity, the nasal plug is disposed to extend upward along the top wall and communicate with the gas cavity.

Optionally, the nasal connector comprises two connecting walls extending outward from two ends of a left end and a right end of the rear wall, respectively, each of the connecting walls is disposed with a via hole along its extending direction, the second part of the first moisture-extraction part is disposed to extend from one end of the first part, extend and penetrate the two connecting walls and then connect with the other end of the first part.

Another aspect of the present application provides a ventilation treatment device, the ventilation treatment device comprises a host as a gas source, a ventilation tube and the patient interface apparatus as mentioned above, and the host is connected with the patient interface apparatus through the ventilation tube.

Optionally, the ventilation treatment device comprises a second moisture-extraction part, wherein the second moisture-extraction part is installed on the ventilation tube and comprises a tube inner part located in the ventilation tube and a tube outer part located outside the ventilation tube, the second moisture-extraction part is disposed to be capable to absorb moisture in the ventilation tube and conduct the moisture to the tube outer part through the tube inner part.

Optionally, a tube wall of the ventilation tube is disposed with an opening, the tube inner part is connected with the inner wall of the ventilation tube, and the tube outer part extends out of the ventilation tube through the opening in a sealed way.

Optionally, the second moisture-extraction part is a long strip shape extending along an axial direction of the ventilation tube, the tube outer part is connected with the outer wall of the ventilation tube.

Optionally, the ventilation treatment device comprises a plurality of second moisture-extraction part, the plurality of second moisture-extraction part are disposed along a circumference of the ventilation tube.

Optionally, the ventilation tube comprises a first tube and a second tube connected with one end of the first tube, one end of the first tube away from one end of the second tube is connected with the patient interface apparatus, one end of the second tube away from one end of the first tube is connected with the host, the tube inner part is connected with the inner wall of the first tube, and the tube outer part is connected with the outer wall of the second tube.

Optionally, the second tube is a heating tube.

Optionally, the second tube is inserted and connected to the first tube, the tube outer part extends to outside of the second tube through an insertion gap between the first tube and the second tube.

Optionally, the second moisture-extraction part has a tubular shape, the outer circumference of the tube inner part is connected to the inner wall of the first tube, the outer wall of the second tube is sleeved in the tube outer part.

Optionally, the second moisture-extraction part is made of fabric or absorbent dressing.

The patient interface apparatus in the present application through disposing the first moisture-extraction part on the main body, it is capable to conduct the moisture in the gas cavity to outside of the main body, which may automatically remove condensed water in the gas cavity, thereby improving safety of ventilation treatment and reducing labor intensity of medical staffs.

Other features and advantages of the present application will be described in detail in the subsequent embodiments as following.

### BRIEF DESCRIPTION OF THE DRAWINGS

The attached figures are configured to provide further understanding of the present application, and being a part of the description and configured to explain the present application with the embodiments below, but do not limit the present application. In the figures:
FIG. 1 is a schematic structural diagram of an embodiment of a patient interface apparatus of the present application, wherein the first moisture-extraction part forms an entire headband;
FIG. 2 is an explosive diagram of FIG. 1;
FIG. 3 is a schematic diagram of the first moisture-extraction part shown in FIG. 1;
FIG. 4 is a longitudinal sectional view of FIG. 2, wherein a second part of the first moisture-extraction part is omitted;
FIG. 5 is a longitudinal sectional view of FIG. 1, wherein the second part of the first moisture-extraction part is omitted;
FIG. 6 is a schematic structural diagram of an embodiment of a ventilation treatment device of the present application, wherein only a patient interface apparatus and a ventilation tube are shown;
FIG. 7 is a partial sectional view of FIG. 6;
FIG. 8 is a schematic structural diagram of another embodiment of the ventilation treatment device of the present application, wherein only the patient interface apparatus and the ventilation tube are shown, the ventilation tube uses the sectional view and a second tube is shown incompletely; and
FIG. 9 is a partial enlargement view of the ventilation tube in FIG. 8.

### Description of labels in the attached figures

10-main body, 101-gas cavity, 11-nasal connector, 111-top wall, 112-rear wall, 113-nasal plug, 114-connecting wall, 115-first flange, 116-second flange, 117-first fixing belt, 118-second fixing belt, 12-adapting connector, 121-shell, 122-top opening, 123-rear opening, 124-interface, 125-first groove, 126-second groove, 20-frist moisture-extraction part, 201-water-absorbing layer, 202-waterproof layer, 21-first part, 22-second part, 30-ventilation tube, 31-first tube, 32-second tube, 40-second moisture-extraction part, 41-tube inner part,
42-tube outer part.

### DETAILED DESCRIPTION OF THE EMBODIMENTS

The following is a detailed description of the embodiments of the present application in combination with the attached figures. It should be understood that the embodiments described herein are intended only to illustrate and explain the present application and are not intended to limit the present application.

In the present application, in the absence of any indication to the contrary, locational terms such as "up, down, top, bottom, left, right, rear" are generally used to refer to the orientation of the patient whom is in a standing state. "Inside and outside" refers to the inside and outside of the parts relative to their own contours.

In one aspect, the present application provides a patient interface apparatus for a ventilation treatment device, wherein the patient interface apparatus includes a main body 10 and a first moisture-extraction part 20, wherein a gas cavity 101 is defined inside the main body 10 and configured to communicate with the patient's mouth and/or nose, the first moisture-extraction part 20 is installed on the main body 10, and it includes a first part 21 connected with the gas cavity 101 and a second part 22 located outside the main body 10, the first moisture-extraction part 20 is disposed to be capable to absorb moisture inside of the gas cavity 101 and conduct the moisture to the second part 22 through the first part 21.

The first part 21 is directly disposed inside the gas cavity 101. In addition, the first moisture-extraction part 20 is an integral part. Moisture in the gas cavity 101 may include water vapor in gas supplied to a patient for inhalation, water vapor in gas exhaled by the patient and condensed water generated in the gas cavity 101.

When using the product, after the first part 21 of the first moisture-extraction part 20 absorbs the moisture in the gas cavity 101, it will diffuse and conduct the moisture to the second part 22, due to the second part 22 is located at external environment of the main body 10, and the external environment humidity is low, relatively dry, so the moisture in the second part 22 is constantly evaporate to the external environment.

The patient interface apparatus of the present application is capable to export the moisture in the gas cavity 101 to the outside of the main body 10 by disposing the first moisture-extraction part 20 on the main body 10, and automatically remove the condensed water in the gas cavity 101, thus improving safety of the ventilation treatment and reducing labor intensity of medical staffs.

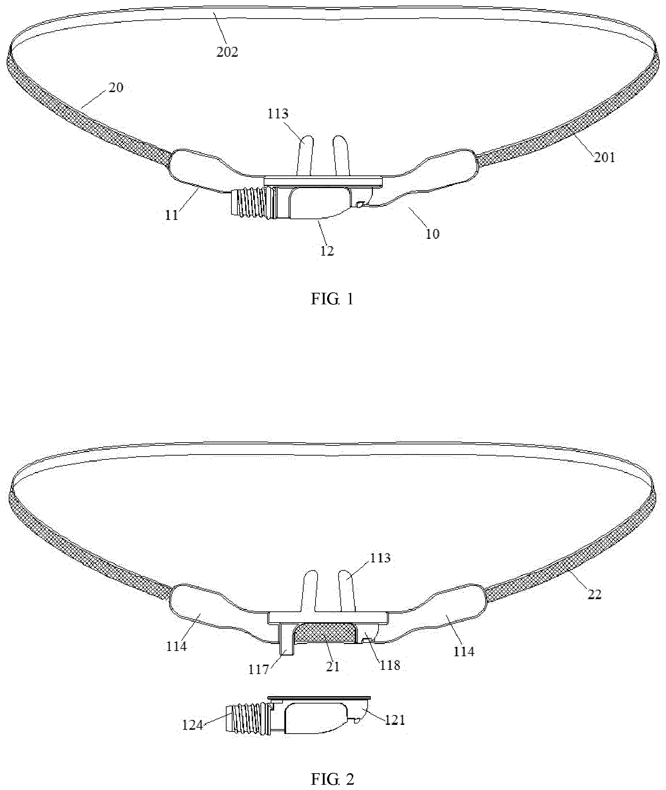

In the present application, the first moisture-extraction part 20 may be an additionally disposed component, or may be disposed as attaching other components of the patient interface apparatus. Due to the patient interface apparatus usually includes a headband configured to fix the main body 10 to the patient's head, therefore, according to an embodiment of the present application, the first moisture-extraction part 20 may be formed as either a part of the headband or an entire headband (see FIG.1-FIG.3)

The headband will contact with the patient's head when the patient interface apparatus is placed on the patient's head. In order to improve the comfort of wearing the apparatus for the patient, the first moisture-extraction part 20 may include a fixed stacked water-absorbing layer 201 and a waterproof layer 202, and the waterproof layer 202 is disposed close to a side of the headband for touching the patient's head. It should be understood that, the first moisture-extraction part 20 with the waterproof layer 202 mentioned above is more suitable for the situation where the first water-extraction part 20 touches the patient's head. In other embodiments, the first water-extraction part 20 may just include the water-absorbing layer 201.

In the present application, the waterproof layer 202 of the first moisture-extraction part 20 may be made of impermeable materials such as plastic or rubber and so forth. In order to improve the comfort of wearing, the waterproof layer 202 is preferably made of softer materials such as silica-gel and so forth. The water-absorbing layer 201 of the first moisture-extraction part 20 may be made of fabric or absorbent dressing, the water-absorbing layer 201 may be single or multi-layer structure. In addition, when the first part 21 is used to limit the gas cavity 101, the water-absorbing layer located in the first part 21 may be preferably provided with a characteristic of good water absorption but high permeability resistance, which may reduce the loss of gas for the patient to inhale, and improve the sealing performance of the gas cavity 101. That is, the first part 21 and the second part 22 of the first water-extraction part 20 may be made of different materials.

In the present application, the patient interface apparatus may be a nasal oxygen catheter or a nasal mask, an oral-nasal mask, a full-face mask and so forth. The present application is further introduced by taking the nasal oxygen catheter as an example.

As referring to FIG. 1, a nasal oxygen catheter is shown, a main body 10 includes a nasal connector 11 and an adapting connector 12 installed on the nasal connector 11, a first moisture-extraction part 20 is installed on the nasal connector 11, a gas cavity 101 is jointly limited formed by the first part 21, the nasal connector 11 and the adapting connector 12, and the adapting connector 12 is capable to pass gas from a gas source into the gas cavity 101.

In the mentions above, it should be understood that, the nasal connector 11 is the part configured to connect with the nasal cavity of the patient; the gas source is an apparatus that produces pressurized air or breathable gas, such as a host of a ventilation treatment device. In addition, the nasal connector 11 may be made of flexible materials (such as silica-gel), the adapting connector 12 may be made of materials harder than the nasal connector 11 (such as plastic) in order to support the nasal connector 11, so that it may ensure that the nasal connector is not easy to deform while comfortable wearing it, thereby ensuring ventilation of the nasal oxygen catheter, and improving reliability of the ventilation of the nasal oxygen catheter.

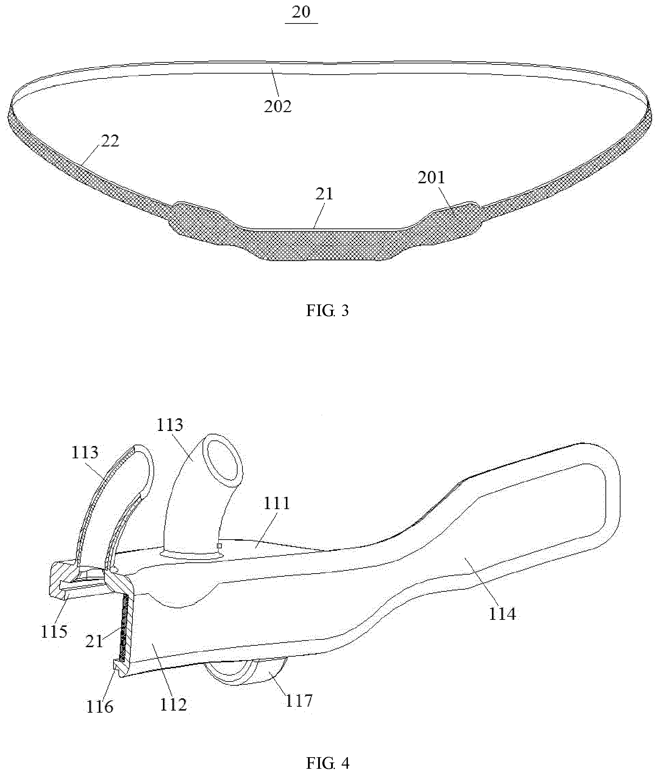

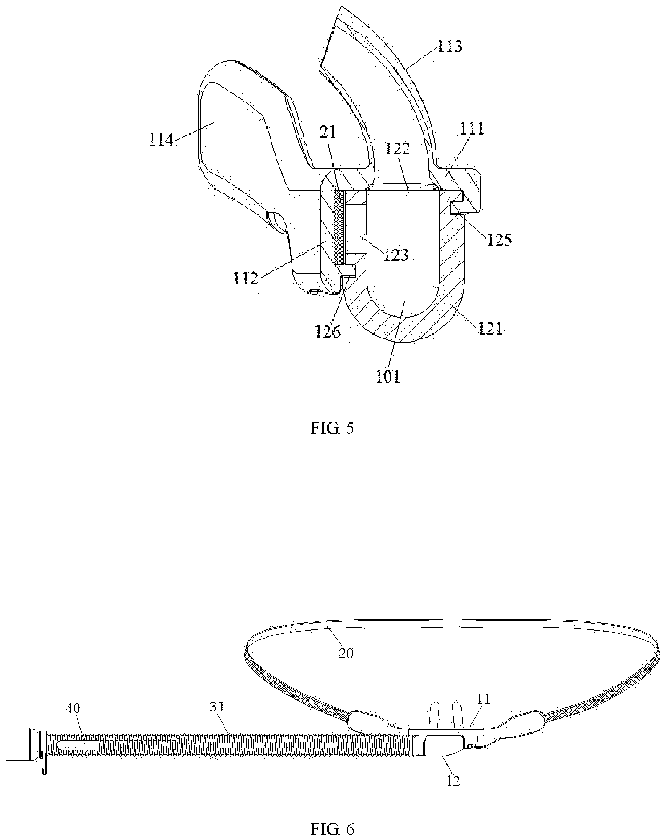

Further, as referring to FIG.4 and FIG. 5, the nasal connector 11 may include an installation structure configured to install the adapting connector 12. The installation structure includes a containing cavity with a bottom opening and a top wall 111 and a rear wall 112 which are configured to limit the containing cavity. The first part 21 is installed on the inner surface of the rear wall 112. The adapting connector 12 includes a shell 121 with a top opening 122 and a rear opening 123. The shell 121 is sealed and installed in the containing cavity via the bottom opening of the installation structure, and the gas cavity 101 is formed by the shell 121, the top wall 111 and the first part 21. In the mentions above, the top wall 111 seals the top opening 122 of the shell 121, the first part 21 seals the rear opening 123 of the shell 121.

In the mentions above, the shell 121 is sealed and installed in the containing cavity, which means that there is no space between the outer wall of the shell 121 and the top wall 111 and the rear wall 112 (as referring to FIG. 5). Further, the shell 121 and the top wall 111 and the rear wall 112 may be clamped through matching structures of flanges and grooves, thereby improving reliability and air tightness of the assembly. Particularly, as referring to FIG.4 and FIG. 5, the top wall 111 is disposed with a first flange 115 which extends downward along the edge of the top wall 111, the outer wall of the shell 121 is disposed with a first groove 125 which is matched with the first flange 115; the rear wall 112 is disposed with a second flange 116 extending forward from the inner surface of the rear wall 112 , the outer wall of the shell 121 is disposed with a second groove 126 which is matched with the second flange 116. In addition, as referring to FIG. 5, an installation groove is formed between the second flange 116 and the top wall 111 which is for the first part 21 embedding in.

In the present application, on the condition that the first moisture-extraction part 20 is formed as a headband, in order to strengthen the connection between the first moisture-extraction 20 and the nasal connector 11, as referring to FIG. 2, the nasal connector 11 may include two connecting walls 114 extending outward from two ends of a left end and a right end of the rear wall 112, respectively. Each of the connecting walls 114 is disposed with a via hole along its extending direction. The second part 22 of the first moisture-extraction part 20 is disposed to extend and pass through the two connecting walls 114 from one end of the first part 21 and then connect with the other end of the first part 21. It should be explained that, during manufacturing, the first moisture-extraction 20 may be molded together with the nasal connector 11.

In addition, as referring to FIG. 4 and FIG. 5, the nasal connector 11 may also include a nasal plug 113 configured to insert a nasal cavity, the nasal plug 113 is disposed to extend upward along the top wall 111 and connect with the gas cavity 101. It should be understood that, quantity of the nasal plug 113 is two.

In the present application, as referring to FIG. 1, the adapting connector 12 may also include an interface 124 configured to connect with a ventilation tube 30 of the gas source, the interface 124 is disposed on the shell 121 and communicate with the gas cavity 101. When using the product, the gas from the gas source passes the ventilation tube 30 and the interface 124 to enter the gas cavity 101, then enters the nasal cavity of the user through the nasal plug 113, through the top opening 122 of the shell 121.

In addition, in order to improve reliability of the assembly of the adapting connector 12 and the nasal connector 11, and to further improve air tightness of the nasal oxygen catheter, the nasal connector 11 may include a fixing part which configured to fix the adapting connector 12 to the installation structure.

Wherein, the fixing part may be a fixing belt of which two ends are connected with the top wall 111 and the rear wall 112, respectively, to form a penetrating space which allows the adapting connector 12 to penetrate. Particularly, as referring to FIG. 1 and FIG. 2, the fixing belt may include a first fixing band 117 and a second fixing band 118 which are interval disposed along the penetrating direction (the direction from left to right as shown in FIG. 2) of the adapting connecter 12. The circumferential profile of the shell 121 may be disposed as decreasing gradually along its penetrating direction. Correspondingly, the penetrating space formed by the first fixing band 117 is larger than that formed by the second fixing band 118. That is, when the adapting connector 12 is installed on the nasal connector 11, the right end of the shell 121 may be easily penetrate the first fixing band 117, and then penetrate inside of the second fixing band 118. The assembly is very simple. In addition, in order to prevent gas leakage from the end being tipped up of the adapting connector 12 which matches the second fixing belt 118, the end of the second fixing band 118 which is away from the first fixing band 117 may be disposed as a closed form (as referring to FIG. 2). At this point, the adapting connector 12 may only be inserted from the left side of the first fixing band 117, but may not be inserted from the right side of the second fixing band 118.

Another aspect of the present application provides a ventilation treatment device, the ventilation treatment device includes the patient interface apparatus as mentioned above.

In the present application, the ventilation treatment device may be a ventilator, a high-flow treatment device and so forth.

The ventilation treatment device may also include a host as the gas source and a ventilation tube 30, the host may connect with the patient interface apparatus through the ventilation tube 30. Particularly, when the patient interface apparatus is a nasal oxygen catheter, one end of the ventilation tube 30 may be connected with a gas outlet of the host, the other end of the ventilation tube 30 may be connected with an interface 124 of the adapting connector 12 of the nasal oxygen catheter.

In the present application, the ventilation treatment device may also include a second moisture-extraction part 40, the second moisture-extraction part 40 is installed on the ventilation tube 30 and includes a tube inner part 41 located in the ventilation tube 30 and a tube outer part 42 located outside the ventilation tube 30, the second moisture-extraction part 40 is disposed to be capable to absorb moisture in the ventilation tube 30 and conduct the moisture to the tube outer part 42 through the tube inner part 41.

When using the product, after the tube inner part 41 of the second moisture-extraction part 40 absorbing the moisture in the ventilation tube 30, it will diffuse and conduct the moisture to the tube outer part 42, due to the tube outer part 42 located at external environment of the ventilation tube 30, and the external environment humidity is low, relatively dry, the moisture in the tube outer part 42 is constantly evaporate to the external environment.

In the present application, by installing the second moisture-extraction part 40 on the ventilation tube 30, condensate water formed in the gas cavity 101 of the patient interface apparatus may flow to the ventilation tube 30, and then be exported by the second moisture-extraction part 40.

Wherein, it should be noted that, the second moisture-extraction part 40 is a similar part to the first moisture-extraction part 20, but since the second moisture-extraction part 40 does not touch the head of the patient, the second moisture-extraction part 40 may only include water-absorbing layer, which may be a single or multi-layer structure made of fabric or absorbent dressing.

In some embodiments, in order to reduce the patient's discomfort caused by gas way dryness and so forth when using the product, the ventilation treatment device may also include a humidifying device configured for heating and humidifying gas, the gas after humidifying may be conveyed to the patient interface apparatus through the ventilation tube 30. Under this condition, the disposing of the second moisture-extraction part 40 may also prevent excessive condensed water formed by humidified gas in the ventilation tube 30 and get into the patient's respiratory tract and cause potentially risks.

According to an embodiment of the ventilation tube 30 in the present application, as referring to FIG. 6, the ventilation tube 30 may include a first tube 31, the second moisture-extraction part 40 may be installed on the first tube 31, the tube inner part 41 of the second moisture-extraction part 40 is located inside of the first tube 31, and the tube outer part 42 of the second moisture-extraction part 40 is located outside of the first tube 31.

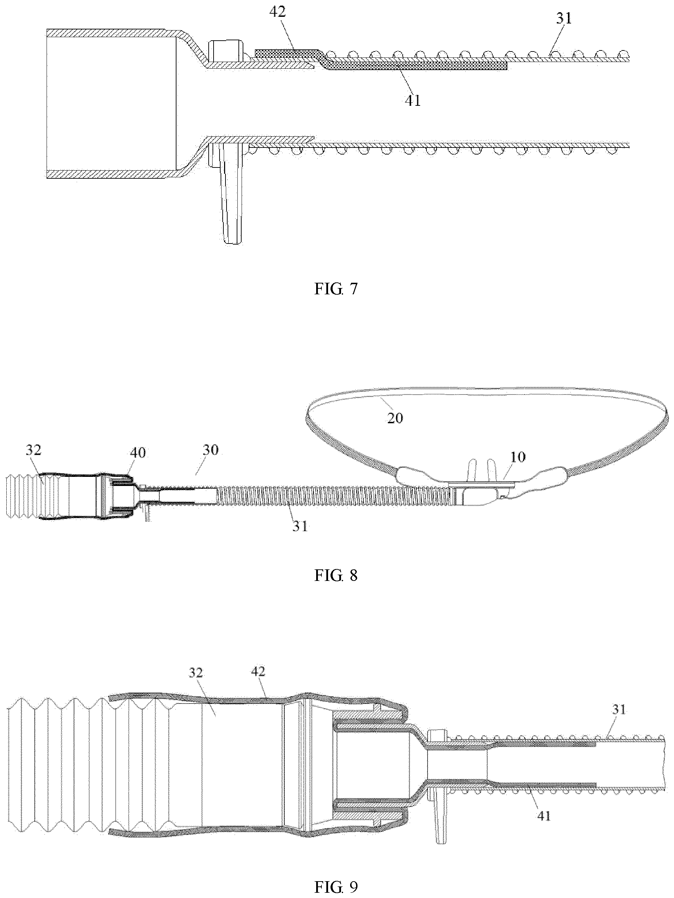

Wherein, for installation of the second moisture-extraction part 40, as referring to FIG. 7, an opening may be disposed on tube wall of the first tube 31, the tube inner part 41 is connected with inner wall of the first tube 31, the tube outer part 42 is sealed by the opening and extends outside the first tube 31. Wherein, the tube inner part 41 of the second moisture-extraction part 40 may be fixed to the inner wall of the first tube 31 by means of bonding or hot-melt welding, the tube outer part 42 may be connected with the outer wall of the first tube 31, or it may not be connected with the outer wall of the first tube 31. In order to improve sealing performance of the first tube 31, during manufacturing, the second moisture-extraction 40 is preferably to be molded together with the first tube 31 to from as an entirety.

In the present application, the second moisture-extraction part 40 may be provided with any shape. Particularly, the implementation way as referring to FIG. 6 and FIG. 7, the second moisture-extraction part 40 may be a long strip shape extending along the axial direction of the first tube 31, the tube outer part 42 is connected with the outer wall of the first tube 31.

Further, in order to improve extraction effect of moisture in the first tube 31, the ventilation tube 30 may include a plurality of second moisture-extraction part 40, the plurality of second moisture-extraction part 40 may be disposed along the circumference of the first tube 31.

The description above introduces the situation of that the ventilation tube 30 includes one tube (that is the first tube 31), in other embodiments of the present application, the ventilation tube 30 may be formed by connecting a plurality of tubes, under this situation, each of the tubes may be installed with the second moisture-extraction part 40, respectively, or the second moisture-extraction part 40 may be installed between two adjacent tubes.

Particularly, the embodiment as referring to FIG. 8, the ventilation tube 30 may also include a second tube 32, the second tube 32 is connected with one end of the first tube 31, the tube inner part 41 of the second moisture-extraction part 40 is connected with the inner wall of the first tube 31, and the tube outer part 42 is connected with the outer wall of the second tube 32.

Further, as referring to FIG. 8 and FIG. 9, the second tube 32 may be inserted to the first tube 31, the tube outer part 42 of the second moisture-extraction part 40, through an insertion space between the first tube 31 and the second tube 32, extends to outside of the second tube 32. Under this situation, the second moisture-extraction part 40 may have a tubular shape, so that the outer circumference surface of the tube inner part 41 is connected to the inner wall of the first tube 31, and the outer wall of the second tube 32 is directly sleeved in the tube outer part 42.

In the mentions above, the second tube 32 may be a heating tube. Under this situation, due to the tube outer part 42 of the second moisture-extraction part 40 touches the outer wall of the second tube 32, so that evaporation of moisture in the tube outer part 42 may be accelerated, thereby improving moisture extraction effect. When using the product, one end of the first tube 31 away from the second tube 32 may be connected with the patient interface apparatus, and one end of the second tube 32 away from the first tube 31 may be connected with the host.

The mentions above combining the attached figures to describe the optimized embodiments specifically, however, the present application is not limited to the specific details of the implementation ways above.

It should also be noted that the specific technical characteristics described in the above specific embodiments may be combined in any appropriate manner without contradiction. In order to avoid unnecessary repetition, the present application does not specify the possible combination ways separately.

In addition, the various embodiments of the present disclosure may also be arbitrarily combined, which shall be deemed to be the content disclosed by the present application, as long as they do not violate the idea of the present application.

## Claims

1. A patient interface apparatus for a ventilation treatment device, wherein the patient interface apparatus comprises a main body (10) and a first moisture-extraction part (20), wherein a gas cavity (101) is defined inside the main body (10) and configured to communicate with the patient's mouth and/or nose, the first moisture-extraction part (20) is disposed on the main body (10) and comprises a first part (21) communicating with the gas cavity (101) and a second part (22) located outside the main body (10), the first moisture-extraction part (20) is configured to be capable of absorbing moisture inside of the gas cavity (101) and conveying the moisture to the second part (22) through the first part (21) directly disposed inside the gas cavity (101),
**characterized in that** the second part (22) is configured to evaporate the moisture to the external environment in use.

2. The patient interface apparatus according to claim 1, the patient interface apparatus comprises a headband, the headband is configured to fix the main body (10) to the patient's head, the first moisture-extraction part (20) is formed as either a part of the headband or the entire headband.

3. The patient interface apparatus according to claim 2, **characterized in that** the moisture-extraction part (20) comprises a fixed stacked water-absorbing layer (201) and a waterproof layer (202), the waterproof layer (202) is disposed close to a side of the headband for touching the patient's head.

4. The patient interface apparatus according to any one of claims 1-3, **characterized in that** the patient interface apparatus is a nasal oxygen catheter, the main body (10) comprises a nasal connector (11) and an adapting connector (12) installed on the nasal connector (11), the first moisture-extraction part (20) is installed on the nasal connector (11), the gas cavity (101) is jointly limited formed by the first part (21), the nasal connector (11) and the adapting connector (12), the adapting connector (12) is capable to pass gas from a gas source to the gas cavity (101).

5. The patient interface apparatus according to claim 4, **characterized in that** the nasal connector (11) comprises an installation structure configured to install the adapting connector (12), the installation structure comprises a containing cavity with a bottom opening and a top wall (111) and a rear wall (112) which are configured to form the containing cavity, the first part (21) is installed on an inner surface of the rear wall (112), the adapting connector (12) comprises a shell (121) with a top opening (122) and a rear opening (123), the shell (121) is sealed and installed in the containing cavity via the bottom opening of the installation structure, the gas cavity (101) is formed by the shell (121), the top wall (111) and the first part (21).

6. The patient interface apparatus according to claim 5, **characterized in that** the nasal connector (11) comprises a nasal plug (113) configured to insert a nasal cavity, the nasal plug (113) is disposed to extend upward along the top wall (111) and communicate with the gas cavity (101); and/or,
the nasal connector (11) comprises two connecting walls (114) extending outward from two ends of a left end and a right end of the rear wall (112), respectively, each of the connecting walls (114) is disposed with a via hole along its extending direction, the second part (22) of the first moisture-extraction part (20) is disposed to extend from one end of the first part (21) and penetrate the two connecting walls (114) and then connect with the other end of the first part (21).

7. A ventilation treatment device, **characterized in that** the ventilation treatment device comprises a host as a gas source, a ventilation tube (30) and the patient interface apparatus according to any one of claims 1-6, and the host is connected with the patient interface apparatus through the ventilation tube (30).

8. The ventilation treatment device according to claim 7 **characterized in that** the ventilation treatment device comprises a second moisture-extraction part (40), wherein the second moisture-extraction part (40) is installed on the ventilation tube (30) and comprises a tube inner part (41) located in the ventilation tube (30) and a tube outer part (42) located outside the ventilation tube (30), the second moisture-extraction part (40) is disposed to be capable to absorb moisture in the ventilation tube (30) and conduct the moisture to the tube outer part (42) through the tube inner part (41).

9. The ventilation treatment device according to claim 8, **characterized in that** a tube wall of the ventilation tube (30) is disposed with an opening, the tube inner part (41) is connected with the inner wall of the ventilation tube (30), and the tube outer part (42) extends out of the ventilation tube (30) through the opening in a sealed way.

10. The ventilation treatment device according to claim 9, **characterized in that** the second moisture-extraction part (40) is a long strip shape extending along an axial direction of the ventilation tube (30), the tube outer part (42) is connected with the outer wall of the ventilation tube (30).

11. The ventilation treatment device according to claim 10, **characterized in that** the ventilation treatment device comprises a plurality of second moisture-extraction part (40), the plurality of second moisture-extraction part (40) are disposed along a circumference of the ventilation tube (30).

12. The ventilation treatment device according to claim 8, **characterized in that** the ventilation tube (30) comprises a first tube (31) and a second tube connected with one end of the first tube (31), one end of the first tube (31) away from one end of the second tube (32) is connected with the patient interface apparatus, one end of the second tube (32) away from one end of the first tube (31) is connected with the host, the tube inner part (41) is connected with the inner wall of the first tube (31), and the tube outer part (42) is connected with the outer wall of the second tube (32).

13. The ventilation treatment device according to claim 12, **characterized in that** the second tube (32) is a heating tube.

14. The ventilation treatment device according to claim 12, **characterized in that**, the second tube (32) is inserted and connected to the first tube (31), the tube outer part (42) extends to outside of the second tube (32) through an insertion gap between the first tube (31) and the second tube (32); and/or
the second moisture-extraction part has a tubular shape, the outer circumference surface of the tube inner part (41) is connected to the inner wall of the first tube (31), the outer wall of the second tube (32) is sleeved in the tube outer part (42).

15. The ventilation treatment device according to any one of claims 9-14, **characterized in that** the second moisture-extraction part (40) is made of fabric or absorbent dressing.

## Patentansprüche

1. Eine Patientenschnittstellenvorrichtung für ein Beatmungsbehandlungsvorrichtung, wobei die Patientenschnittstellenvorrichtung einen Hauptkörper (10) und ein erstes Feuchtigkeitsextraktionsteil (20) umfasst, wobei ein Gashohlraum (101) innerhalb des Hauptkörpers (10) definiert und so konfiguriert ist, dass er mit dem Mund und/oder der Nase des Patienten in Verbindung steht, wobei das erste Feuchtigkeitsextraktionsteil (20) auf dem Hauptkörper (10) angeordnet ist und ein ersten Teil (21), der mit dem Gashohlraum (101) in Verbindung steht, und einen zweiten Teil (22), der außerhalb des Hauptkörpers (10) angeordnet ist, umfasst, wobei der erste Feuchtigkeitsextraktionsteil (20) so konfiguriert ist, dass er Feuchtigkeit im Inneren des Gashohlraums (101) absorbieren und die Feuchtigkeit durch den ersten Teil (21), der direkt im Inneren des Gashohlraums (101) angeordnet ist, zum zweiten Teil (22) befördern kann,
**dadurch gekennzeichnet, dass** der zweite Teil (22) so konfiguriert ist, dass er die Feuchtigkeit bei Gebrauch an die äußere Umgebung verdampft.

2. Die Patientenschnittstellenvorrichtung gemäß Anspruch 1, wobei die Patientenschnittstellenvorrichtung ein Stirnband umfasst, wobei das Stirnband so konfiguriert ist, dass es den Hauptkörper (10) am Kopf des Patienten befestigt, wobei der erste Feuchtigkeitsextraktionsteil (20) entweder als Teil des Stirnbands oder des gesamten Stirnbands ausgebildet ist.

3. Die Patientenschnittstellenvorrichtung gemäß Anspruch 2, **dadurch gekennzeichnet, dass** der Feuchtigkeitsextraktionsteil (20) eine fest angeordnete, gestapelte, wasserabsorbierende Schicht (201) und eine wasserdichte Schicht (202) umfasst, wobei die wasserdichte Schicht (202) nahe einer Seite des Kopfbands angeordnet ist, um den Kopf des Patienten zu berühren.

4. Die Patientenschnittstellenvorrichtung gemäß einem der Ansprüche 1-3, **dadurch gekennzeichnet, dass** die Patientenschnittstellenvorrichtung ein nasaler Sauerstoffkatheter ist, der Hauptkörper (10) einen Nasenanschluss (11) und einen an dem Nasenanschluss (11) angebrachten Anpassungsanschluss (12) umfasst, das erste Feuchtigkeitsextraktionsteil (20) ist an dem Nasenanschluss (11) angebracht, der Gashohlraum (101) ist durch das erste Teil (21), den Nasenanschluss (11) und den Anpassungsanschluss (12) gemeinsam begrenzt ausgebildet, der Anpassungsanschluss (12) ist in der Lage, Gas von einer Gasquelle zu dem Gashohlraum (101) zu leiten.

5. Die Patientenschnittstellenvorrichtung gemäß Anspruch 4, **dadurch gekennzeichnet, dass** der Nasenanschluss (11) eine Installationsstruktur umfasst, die so konfiguriert ist, dass sie den Anpassungsanschluss (12) installiert, wobei die Installationsstruktur einen Aufnahmeraum mit einer Bodenöffnung und einer oberen Wand (111) und einer Rückwand (112) umfasst, die so konfiguriert sind, dass sie den Aufnahmeraum bilden, wobei der erste Teil (21) an einer Innenfläche der Rückwand (112) installiert ist, der Anpassungsanschluss (12) eine Schale (121) mit einer oberen Öffnung (122) und einer hinteren Öffnung (123) umfasst, die Schale (121) abgedichtet und in dem aufnehmenden Hohlraum über die Bodenöffnung der Installationsstruktur installiert ist, der Gashohlraum (101) durch die Schale (121), die obere Wand (111) und den ersten Teil (21) gebildet wird.

6. Die Patientenschnittstellenvorrichtung nach Anspruch 5, **dadurch gekennzeichnet, dass** der Nasenanschluss (11) einen Nasenstöpsel (113) umfasst, der so konfiguriert ist, dass er in eine Nasenhöhle eingeführt werden kann, wobei der Nasenstöpsel (113) so angeordnet ist, dass er sich entlang der oberen Wand (111) nach oben erstreckt und mit dem Gasraum (101) in Verbindung steht; und/oder
der Nasenanschluss (11) zwei Verbindungswände (114) umfasst, die sich von zwei Enden eines linken Endes bzw. eines rechten Endes der Rückwand (112) nach außen erstrecken, wobei jede der Verbindungswände (114) mit einem Durchgangsloch entlang ihrer Erstreckungsrichtung versehen ist, der zweite Teil (22) des ersten Feuchtigkeitsextraktionsteils (20) so angeordnet ist, dass er sich von einem Ende des ersten Teils (21) erstreckt und die beiden Verbindungswände (114) durchdringt und dann mit dem anderen Ende des ersten Teils (21) verbunden ist.

7. Eine Beatmungsbehandlungsvorrichtung, **dadurch gekennzeichnet, dass** die Beatmungsbehandlungsvorrichtung einen Wirt als Gasquelle, einen Beatmungsschlauch (30) und die Patientenschnittstellenvorrichtung gemäß einem der Ansprüche 1 bis 6 umfasst und der Wirt mit der Patientenschnittstellenvorrichtung über den Beatmungsschlauch (30) verbunden ist.

8. Die Beatmungsbehandlungsvorrichtung nach Anspruch 7, **dadurch gekennzeichnet, dass** die Beatmungsbehandlungsvorrichtung ein zweites Feuchtigkeitsextraktionsteil (40) umfasst, wobei das zweite Feuchtigkeitsextraktionsteil (40) an dem Beatmungsschlauch (30) angebracht ist und ein Schlauchinnenteil (41), das sich in dem Beatmungsschlauch (30) befindet und ein Schlauchaußenteil (42), das außerhalb des Beatmungsschlauchs (30) angeordnet ist, umfasst, wobei das zweite Feuchtigkeitsextraktionsteil (40) so angeordnet ist, dass es Feuchtigkeit in dem Beatmungsschlauch (30) absorbieren und die Feuchtigkeit durch das Schlauchinnenteil (41) zu dem Schlauchaußenteil (42) leiten kann.

9. Die Beatmungsbehandlungsvorrichtung nach Anspruch 8, **dadurch gekennzeichnet, dass** eine Schlauchwand des Beatmungsschlauchs (30) mit einer Öffnung versehen ist, der Rohrinnenteil (41) mit der Innenwand des Beatmungsschlauchs (30) verbunden ist und der Schlauchaußenteil (42) sich durch die Öffnung abgedichtet aus dem Beatmungsschlauch (30) heraus erstreckt.

10. Die Beatmungsbehandlungsvorrichtung nach Anspruch 9, **dadurch gekennzeichnet, dass** der zweite Feuchtigkeitsextraktionsteil (40) eine lange Streifenform ist, die sich entlang einer axialen Richtung des Beatmungsschlauchs (30) erstreckt, wobei der Schlauchaußenteil (42) mit der Außenwand des Beatmungsschlauchs (30) verbunden ist.

11. Die Beatmungsbehandlungsvorrichtung gemäß Anspruch 10, **dadurch gekennzeichnet, dass** die Beatmungsbehandlungsvorrichtung eine Vielzahl von zweiten Feuchtigkeitsextraktionsteilen (40) umfasst, wobei die Vielzahl von zweiten Feuchtigkeitsextraktionsteilen (40) entlang eines Umfangs des Beatmungsschlauchs (30) angeordnet sind.

12. Die Beatmungsbehandlungsvorrichtung nach Anspruch 8, **dadurch gekennzeichnet, dass** der Beatmungsschlauch (30) einen ersten Schlauch (31) und einen zweiten Schlauch umfasst, der mit einem Ende des ersten Schlauchs (31) verbunden ist, wobei ein Ende des ersten Schlauchs (31), das von einem Ende des zweiten Schlauchs (32) entfernt ist, mit der Patientenschnittstellenvorrichtung verbunden ist, ein Ende des zweiten Schlauchs (32), das von einem Ende des ersten Schlauchs (31) entfernt ist, mit dem Wirt verbunden ist, der Schlauchinnenteil (41) mit der Innenwand des ersten Schlauchs (31) verbunden ist und der Schlauchaußenteil (42) mit der Außenwand des zweiten Schlauchs (32) verbunden ist.

13. Die Beatmungsbehandlungsvorrichtung nach Anspruch 12, **dadurch gekennzeichnet, dass** der zweite Schlauch (32) ein Heizschlauch ist.

14. Die Beatmungsbehandlungsvorrichtung nach Anspruch 12, **dadurch gekennzeichnet, dass** der zweite Schlauch (32) in den ersten Schlauch (31) eingesetzt und mit diesem verbunden ist, wobei sich der Schlauchaußenteil (42) durch einen Einführspalt zwischen dem ersten Schlauch (31) und dem zweiten Schlauch (32) zur Außenseite des zweiten Schlauchs (32) erstreckt; und/oder
der zweite Feuchtigkeit entziehende Teil eine röhrenförmige Gestalt hat, die Außenumfangsfläche des röhrenförmigen Innenteils (41) mit der Innenwand des ersten Schlauchs (31) verbunden ist, die Außenwand des zweiten Schlauchs (32) in den Schlauchaußenteil (42) hülsenartig eingesetzt ist.

15. Die Beatmungsbehandlungsvorrichtung nach einem der Ansprüche 9-14, **dadurch gekennzeichnet, dass** der zweite Feuchtigkeitsextraktionsteil (40) aus Stoff oder einem absorbierenden Verband besteht.

## Revendications

1. Appareil d'interface patient pour un dispositif de traitement par ventilation, dans lequel l'appareil d'interface patient comprend un corps principal (10) et une première partie d'extraction d'humidité (20), dans lequel une cavité de gaz (101) est définie à l'intérieur du corps principal (10) et configurée pour communiquer avec la bouche et/ou le nez du patient, la première partie d'extraction d'humidité (20) est disposée sur le corps principal (10) et comprend une première partie (21) communiquant avec la cavité de gaz (101) et une seconde partie (22) localisée à l'extérieur du corps principal (10), la première partie d'extraction d'humidité (20) est configurée pour pouvoir absorber l'humidité à l'intérieur de la cavité de gaz (101) et transporter l'humidité vers la seconde partie (22) à travers la première partie (21) directement disposée à l'intérieur de la cavité de gaz (101),
**caractérisé en ce que** la seconde partie (22) est configurée pour évaporer l'humidité vers l'environnement externe durant l'utilisation.

2. Appareil d'interface patient selon la revendication 1, l'appareil d'interface patient comprend un bandeau, le bandeau est configuré pour fixer le corps principal (10) à la tête du patient, la première partie d'extraction d'humidité (20) est formée soit comme une partie du bandeau soit comme le bandeau entier.

3. Appareil d'interface patient selon la revendication 2, **caractérisé en ce que** la partie d'extraction d'humidité (20) comprend une couche (201) absorbant l'eau empilée de manière fixe et une couche étanche à l'eau (202), la couche étanche à l'eau (202) est disposée proche d'un côté du bandeau pour toucher la tête du patient.

4. Appareil d'interface patient selon l'une quelconque des revendications 1 à 3, **caractérisé en ce que** l'appareil d'interface patient est un cathéter d'oxygène nasal, le corps principal (10) comprend un raccord nasal (11) et un raccord d'adaptation (12) installé sur le raccord nasal (11), la première partie d'extraction d'humidité (20) est installée sur le raccord nasal (11), la cavité de gaz (101) est formée limitée de manière conjointe par la première partie (21), le raccord nasal (11) et le raccord d'adaptation (12), le raccord d'adaptation (12) est capable de faire passer du gaz depuis une source de gaz vers la cavité de gaz (101).

5. Appareil d'interface patient selon la revendication 4, **caractérisé en ce que** le raccord nasal (11) comprend une structure d'installation configurée pour installer le raccord d'adaptation (12), la structure d'installation comprend une cavité de confinement avec une ouverture inférieure et une paroi supérieure (111) et une paroi arrière (112) qui sont configurées pour former la cavité de confinement, la première partie (21) est installée sur une surface interne de la paroi arrière (112), le raccord d'adaptation (12) comprend une coque (121) avec une ouverture supérieure (122) et une ouverture arrière (123), la coque (121) est scellée et installée dans la cavité de confinement par l'intermédiaire de l'ouverture inférieure de la structure d'installation, la cavité de gaz (101) est formée par la coque (121), la paroi supérieure (111) et la première partie (21).

6. Appareil d'interface patient selon la revendication 5, **caractérisé en ce que** le raccord nasal (11) comprend un bouchon nasal (113) configuré pour l'insertion dans une cavité nasale, le bouchon nasal (113) est disposé pour s'étendre vers le haut le long de la paroi supérieure (111) et pour communiquer avec la cavité de gaz (101) ; et/ou,
le raccord nasal (11) comprend deux parois de raccordement (114) s'étendant vers l'extérieur depuis deux extrémités d'une extrémité gauche et d'une extrémité droite de la paroi arrière (112), respectivement, chacune des parois de raccordement (114) est disposée par l'intermédiaire d'un trou traversant, le long de son sens d'extension, la seconde partie (22) de la première partie d'extraction d'humidité (20) est disposée pour s'étendre depuis une extrémité de la première partie (21) et pour pénétrer les deux parois de raccordement (114) et ensuite se raccorder à l'autre extrémité de la première partie (21).

7. Dispositif de traitement par ventilation, **caractérisé en ce que** le dispositif de traitement par ventilation comprend un hôte comme source de gaz, un tube de ventilation (30) et l'appareil d'interface patient selon l'une quelconque des revendications 1 à 6, et l'hôte est raccordé à l'appareil d'interface patient à travers le tube de ventilation (30).

8. Dispositif de traitement par ventilation selon la revendication 7, **caractérisé en ce que** le dispositif de traitement par ventilation comprend une seconde partie d'extraction d'humidité (40), dans lequel la seconde partie d'extraction d'humidité (40) est installée sur le tube de ventilation (30) et comprend une partie interne de tube (41) localisée dans le tube de ventilation (30) et une partie externe de tube (42) localisée à l'extérieur du tube de ventilation (30), la seconde partie d'extraction d'humidité (40) est disposée pour pouvoir absorber l'humidité dans le tube de ventilation (30) et conduire l'humidité vers la partie externe de tube (42) à travers la partie interne de tube (41).

9. Dispositif de traitement par ventilation selon la revendication 8, **caractérisé en ce qu'**une paroi de tube du tube de ventilation (30) est disposée avec une ouverture, la partie interne de tube (41) est raccordée à la paroi interne du tube de ventilation (30), et la partie externe de tube (42) s'étend hors du tube de ventilation (30) à travers l'ouverture d'une manière scellée.

10. Dispositif de traitement par ventilation selon la revendication 9, **caractérisé en ce que** la seconde partie d'extraction d'humidité (40) est une longue forme de bande s'étendant le long d'un sens axial du tube de ventilation (30), la partie externe de tube (42) est raccordée à la paroi externe du tube de ventilation (30).

11. Dispositif de traitement par ventilation selon la revendication 10, **caractérisé en ce que** le dispositif de traitement par ventilation comprend une pluralité de seconde partie d'extraction d'humidité (40), la pluralité de seconde partie d'extraction d'humidité (40) sont disposées le long d'une circonférence du tube de ventilation (30).

12. Dispositif de traitement par ventilation selon la revendication 8, **caractérisé en ce que** le tube de ventilation (30) comprend un premier tube (31) et un second tube raccordé à une extrémité du premier tube (31), une extrémité du premier tube (31) à l'opposé d'une extrémité du second tube (32) est raccordée à l'appareil d'interface patient, une extrémité du second tube (32) à l'opposé d'une extrémité du premier tube (31) est raccordée à l'hôte, la partie interne de tube (41) est raccordée à la paroi interne du premier tube (31), et la partie externe de tube (42) est raccordée à la paroi externe du second tube (32).

13. Dispositif de traitement par ventilation selon la revendication 12, **caractérisé en ce que** le second tube (32) est un tube chauffant.

14. Dispositif de traitement par ventilation selon la revendication 12, **caractérisé en ce que**, le second tube (32) est inséré et raccordé au premier tube (31), la partie externe de tube (42) s'étend à l'extérieur du second tube (32) à travers un espace d'insertion entre le premier tube (31) et le second tube (32) ; et/ou
la seconde partie d'extraction d'humidité présente une forme tubulaire, la surface de circonférence externe de la partie interne de tube (41) est raccordée à la paroi interne du premier tube (31), la paroi externe du second tube (32) est emmanchée dans la partie externe de tube (42).

15. Dispositif de traitement par ventilation selon l'une quelconque des revendications 9 à 14, **caractérisé en ce que** la seconde partie d'extraction d'humidité (40) est constituée de textile ou de pansement absorbant.
